# EUROPEAN PATENT APPLICATION

(11) **EP 2 462 927 A1**
(43) Date of publication of application: **13.06.2012**
(21) Application number: 10015269.3
(22) Date of filing: 03.12.2010
(51) Int. Cl.: A61K 9/70, A61K 31/4468, A61P 25/04

(54) **Transdermal therapeutic system comprising fentanyl**

(71) Applicant: Hexal AG, 83607 Holzkirchen (DE)
(72) Inventor: Fleschhut, Jens, Dr., 83607 Holzkirchen (DE); Feinäugle, Susanne, 83607 Holzkirchen (DE); Lauer, Karin, Dr., 83607 Holzkirchen (DE)
(74) Representative: Aechter, Bernd

(57) **Abstract**

The invention is concerned with a transdermal therapeutic system (TTS) comprising fentanyl and a method of manufacturing such a TTS. The transdermal therapeutic system is used for the transdermal administration of fentanyl and analogues thereof. In particular, the invention relates to the use of a transdermal therapeutic system (TTS) for analgesic purposes. The TTS according to the invention comprises a drug containing adhesive composition comprising fentanyl, a styrenic polymer, a liquid diluent, a penetration enhancer and optionally a tackifier.

## Description

The invention is concerned with a transdermal therapeutic system (TTS) for the transdermal administration of fentanyl and analogues thereof and further relates to a method for producing such a TTS. In particular, the invention relates to the use of a transdermal therapeutic system for analgetic purposes.

### General remarks:

Fentanyl and analogues thereof are strong agonist of opiate receptors. They have widely been used due to its effectiveness in relieving pain as potent narcotic analgesics in the treatment of chronic pain, in particular for treating post-operative pain, in cancer therapy and other chronic pain management. Through the delivery method of transdermal patches, fentanyl is currently the most widely used synthetic opioid in clinical practice.

The use of fentanyl in treating pain, however, has the disadvantage that its administration is connected with a rapid onset and short duration of action. To control the delivery of fentanyl and to enable a constant release of the drug to the patient, transdermal matrix systems have been shown to be particularly useful for fentanyl administration.

Transdermal delivery systems and more specifically transdermal therapeutic systems which are also referred to herein as TTS, such as transdermal patches, have been proven to be advantageous in the administration and delivery of pharmaceutically active agents, such as fentanyl. One of the reasons for this is that transdermal delivery systems avoid hepatic metabolization of the pharmaceutically active agent, which is frequently observed upon oral administration of a pharmaceutically active agent. As a consequence, upon administration of a pharmaceutically active agent through a transdermal delivery system the liver is relieved and gastrointestinal side effects are avoided. Additionally, compared to a non-transdermal administration, usually less of the pharmaceutically active agent is required so as to have the same effect. Furthermore, transdermal delivery systems provide a more constant blood level of the pharmaceutically active agent as said agent is immediately effective in a systemic manner upon permeation through the skin. Finally, transdermal delivery systems increase patients' compliance due to their easy and convenient application.

Common transdermal delivery systems have the disadvantage that they often do not provide sufficient adhesive power to remain attached to a patient's skin for the period of time needed for administration of the drug, which can be from three to seven days. Thus, a need exists for alternative pressure sensitive adhesive polymers for use in transdermal applications.

Commercially available classes of adhesives for use in skin contact applications such as TTS are acrylic polymers, polyisobutylene polymers (PIB) and silicone polymers. However, styrenic polymers have not yet been commercially used due to a widely-held prejudice that copolymers with high styrene content make the polymer difficult to tackify (Istvan Benedek; Pressure-Sensitive Adhesives and Applications, 2nd edition, 2004).

As fentanyl is a strong opioid analogue, the use of this analgesic bears a high abusive risk. The disadvantage of a transdermal reservoir system such as a transdermal patch is that its abusive use is easily possible due to reuse of the liquid reservoir content. The abusive, non-medical use of fentanyl by individuals without opiate tolerance can be very dangerous and can even result in death of the individual. Because the effects of fentanyl last for only a very short time, it is even more addictive than heroin, and regular users may become addicted very quickly. Even matrix systems could be abused, wherein a higher drug load of the matrix system leads to a higher risk of drug abuse. Therefore, a need exists for a fentanyl application with reduced risk of abuse. This could be achieved by providing low-dosage fentanyl applications including only a low amount of drug.

Fentanyl as potent opioid, however, has a relatively narrow therapeutic index. As a consequence, the therapeutic effect is obtained only over a narrow range of concentrations. Administration of a fentanyl concentration below a specific range is ineffective in relieving pain and concentrations above the effective range are associated with serious and dangerous side effects.

Accordingly, application of a low-dosage fentanyl patch bears the risk that either the patch releases not enough active drug to relieve pain in the patient, or the amount of drug contained in the transdermal patch is completely released to the patient in a short period of time, making it necessary to apply new patches after a relatively short time.

WO 03/074035 A1 describes a transdermal system containing fentanyl as the active ingredient, a resin, an adhesive and an oil-based aloe vera extract. This application, however, does not describe the provision of a low-dosage fentanyl patch for the constant release of drug for more than three days, which, at the same time, provides adequate adhesive power to ensure adhesion to the skin for the time needed to release the drug.

WO 02/074286 A1 relates to a transdermal patch for administering fentanyl and analogues thereof for analgesic purposes. Low dosage fentanyl is disclosed in this application only in combination with an acrylic polymer adhesive.

The combination of potential drug abuse and narrow therapeutic index thus makes it extremely difficult to provide a fentanyl transdermal system such as a transdermal patch having a low drug content but releasing an appropriate amount of the drug to the patient for reliving pain for the longest possible period.

Thus, a need exists for a low dosage transdermal fentanyl system which releases an effective amount of drug for relieving pain in a patient in need of an analgesic and which is effective in providing a constant drug level in the patient which is effective in relieving pain for three days or more.

### Object of the invention:

Thus, it is the object of the present invention to provide a transdermal therapeutic system (TTS) containing a drug delivery composition for use in a TTS which solves the aforementioned problems. In particular, it is the object of the present invention to provide a transdermal system which has a low risk of abuse and which releases an effective amount of drug for relieving pain in a patient in need of an analgesic for more than three days. The object is further to provide a transdermal system which has strong adhesive power and which is effective in providing a constant drug level in the patient which is effective in relieving pain for three days or more.

### Description of the drawings:

**Figure 1** shows the in vitro skin permeation of a fentanyl TTS according to the invention compared to a reference system.
**Figure 2** shows the blood plasma concentration of fentanyl in in vivo studies compared to a reference system.

### Description of the invention:

The inventors have surprisingly found that a TTS for the administration of a low amount of fentanyl comprising an adhesive composition of a styrenic polymer solves the above problems.

Such a TTS is particularly useful for providing a constant and high blood plasma level of fentanyl and continuous drug delivery for three days or more (≥ 72 h), preferably four days or more (≥ 96 h), most preferably for four days (about 96 h), while at the same time providing optimum adhesive power and greatest comfort to the patient for the entire period of use.

The above-mentioned objects have thus been surprisingly solved in the present invention by the provision of a transdermal therapeutic system comprising
1) optionally a backing layer,
2) a drug containing adhesive composition,
3) optionally one or more adhesive layers, and
4) further optionally a release liner,
   wherein the drug containing adhesive composition comprises
   a) 0.1 to 6.0% by weight of fentanyl base, based on the total amount of the composition,
   b) a styrenic polymer, preferably a styrenic copolymer,
   c) a liquid diluent,
   d) a penetration enhancer, and
   e) optionally a tackifier.

The present invention further relates to method of producing a transdermal therapeutic system comprising a backing layer, at least one adhesive layer and a release liner, comprising the steps of
1) preparing a solution or dispersion comprising
   a. a fentanyl base,
   b. a styrenic polymer, preferably a styrenic copolymer, and
   c. optionally one or more of a penetration enhancer, liquid diluent or tackifier,
2) coating the solution or dispersion on the backing layer or on the release liner,
3) drying the solution or dispersion to form the at least one adhesive layer, and
4) coating the at least one adhesive layer with the release liner or the backing layer.

The invention further relates to a TTS, which is obtainable by the method of the present invention.

The TTS of the present invention is particularly useful for treating a patient suffering from acute or chronic pain.

### Detailed description of the invention:

The present invention provides a TTS for the transdermal delivery of fentanyl. The TTS of the invention typically is a multi-layered system comprising at least one drug containing adhesive layer. The drug containing adhesive layer comprises or consists of a drug containing adhesive composition comprising fentanyl or fentanyl analogue as active ingredient. The drug containing adhesive composition further comprises an adhesive component of styrenic polymer.

### Fentanyl:

Fentanyl (component a)) relates to a compound represented by the following formula: 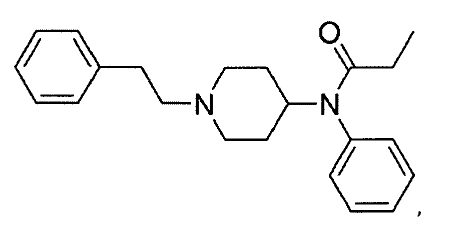 which has the IUPAC nomenclature of N-(1-(2-phenylethyl)-4-piperidinyl)-N-phenylpropanamide.

The term "fentanyl" in the context of the present invention comprises fentanyl and analogues thereof such as a fentanyl salt or fentanyl in its basic form. In preferred embodiments, the drug used in the transdermal system of the present invention is fentanyl in the basic form (fentanyl base). Fentanyl salts include, but are not limited to, salts such as the hydrochloride, sulfate, phosphate or its various organic acid salts such as maleate, succinate, mesylate and tosylate.

The present invention is particularly concerned with TTS comprising low-dosage fentanyl. Thus, fentanyl can be contained in the drug containing adhesive composition of the present invention in an amount of 0.1 to 6 % by weight, preferably in an amount of 1 to 5 % by weight, based on the total amount of the drug containing adhesive composition. In more preferred embodiments, fentanyl is contained in the drug containing adhesive composition in an amount of 2 to 4.5, even more preferably, in an amount of 2.5 to 4 % by weight, based on the total amount of the drug containing adhesive composition.

An amount of below 0.1 % by weight of fentanyl is usually not sufficient to induce analgesia whereas an amount of above 6 % may increase the risk of drug abuse due to the high concentration of the drug in the adhesive layer.

### Styrenic polymer:

The TTS of the present invention comprises at least one drug containing adhesive layer, which is formed from a drug containing adhesive composition. The drug containing adhesive composition comprises an adhesive in the form of a styrenic polymer (component b)). Preferably, the styrenic polymer is a styrenic copolymer, most preferably a block copolymer.

The styrenic polymer such as the styrenic copolymer can be a linear, random, graft or block polymer. Preferred block copolymers are di- and triblock copolymers, such as A-B, A-B-A and A-B-C block copolymers.

In a preferred embodiment, a single block of the block copolymer of the invention has a weight average molecular weight of from 100 to 10,000,000, preferably from 1,000 to 1,000,000, most preferably from 2,000 to 200,000 as measured by gel permeation chromatography.

In a preferred embodiment of the invention the styrenic copolymer is an A-B-A triblock copolymer. More preferably, the A-B-A copolymer comprises at least one styrenic polymer block A, preferably polystyrene, and at least one polymer block B obtained from monomers selected from ethylene, propylene, isoprene, butylene, butadiene or mixtures thereof.

The A-block may further comprise from 1 to 95 wt.%, preferably from 5 to 50 wt. %, based on the total weight of the A-blocks of the styrene copolymer, of homopolymers or copolymers of vinyl monomers such as vinyl arenes, vinyl pyridines, vinyl halides and vinyl carboxylates. Monovinyl aromatic hydrocarbons include particularly those of the benzene series such as styrene, vinyl toluene, vinyl xylene, ethyl vinyl benzene as well as dicyclic monovinyl compounds such as vinyl naphthalene and the like. Other polymer blocks, which may be contained in the A-block of the styrene copolymer, may be derived from alpha olefins, alkylene oxides, acetals, and urethanes. Styrene is preferred.

The polymer block B can be obtained from isoprene, butadiene or mixtures thereof. Moreover, when the polymer block B is obtained from one of these monomers, the polymer block B can be completely or partially hydrogenated and preferably more than 50 % of the unsaturated, hydrogenizable bondings are hydrogenated.

In a preferred embodiment the end-block portion (i.e., the "A-block") of the styrenic copolymer has a glass transition temperature T_{g} which is higher than the glass transition temperature of the mid-block portion (i.e., the "B-block"). Preferably, the glass transition temperature T_{g} of the A-block is from 0 to 200 °C, more preferably from 20 to 150 °C, most preferably from 30 to 120 °C. Glass transition measurement is achieved by ISO 11357-1, -2, -3.

In a further preferred embodiment, the B-block of the styrenic copolymer has a glass transition temperature Tg less than about room temperature. Preferably, the Tg is in the range of -30 to 20 °C, preferably from -25 °C to 10 °C, as measured by ISO 11357-1, -2, -3. The A- and B- blocks can be contained in the styrenic copolymer in a ratio of 5 : 95 to 95 : 5, preferably 10 : 95 to 40 : 60.

In an even more preferred embodiment, the A-B-A triblock copolymer is selected from styrene-ethylene-styrene (SES), styrene-butadiene-styrene (SBS), styrene-isoprene-styrene (SIS), styrene-ethylene/butylene-styrene (S-EB-S), styrene-ethylene/butylene/propylene-styrene (S-EBP-S), styrene-isoprene/butadiene-styrene (S-IB-S), or mixtures thereof. In this description the hyphen "-" is used for separating the A- from the B-blocks, whereas a slash "/" is used for separating individual monomers in case the B-block is obtained from more than one monomer.

For the A-B-A triblock copolymer, the polymer block B can be completely or partially hydrogenated and preferably more than 50 % of the unsaturated, hydrogenizable bondings are hydrogenated.

The drug containing adhesive composition may further comprise from 1 to 95 wt.%, preferably from 5 to 55 wt.%, based on the total amount of the styrenic polymers contained in the drug containing adhesive composition, of a styrenic A-B diblock copolymer. In this embodiment, blocks A and B may be defined as above for the styrenic triblock copolymer. The styrenic diblock copolymer is preferably selected from styrene-ethylene (SE), styrene-butadiene (SB), styrene-isoprene (SI), styrene-ethylene/butadiene-styrene (SE-BS), styrene-ethylene/propylene (S-EP), or mixtures thereof.

For the A-B diblock copolymer, the polymer block B can be completely or partially hydrogenated and preferably more than 50 % of the unsaturated, hydrogenizable bondings are hydrogenated.

In a preferred embodiment, styrenic polymer of the invention has a weight average molecular weight of from 1000 to 10,000,000, preferably from 5,000 to 2,000,000, most preferably from 10,000 to 500,000 as measured by gel permeation chromatography.

It is preferred that the adhesive composition additionally comprises up to 20, preferably 3 to 8, parts by weight of a high molecular weight (i.e., viscosity of 1,000 to 1,000,000 cps as measured at 25 °C. at 20% in toluene) diblock polymer of the general A-B configuration. In this diblock copolymer, the A- and B-blocks are generally as described above. While the adhesive composition preferably comprises a diblock polymer, the diblock may be replaced entirely or in part with another high molecular weight polymer such as polyisobutylene and/or polyisoprene. The high molecular weight polymer may be used in amounts of 1 to 15 wt.%, preferably 2 to 10 wt.% based on the total drug containing adhesive composition.

In a preferred embodiment the styrenic polymer b) comprises a hydrogenated styrene-isoprene/butadiene-styrene block copolymer (S-IB-S) and a hydrogenated styrene-isoprene block copolymer (SI).

### Liquid diluent:

The drug containing adhesive composition may further comprise one or more of a liquid diluent (component c)). The liquid diluent may have the function of a plasticizer for reducing the glass transition temperature (T_{g}) of the mid-block polymer (B-block). Thus, these components may associate with the B-blocks described above by swelling it, which generally results in a change, preferably in a decrease in the Tg thereof.

In a preferred embodiment, the liquid diluent is an oil component such as mineral oil, preferably paraffinic mineral oil, naphthenic mineral oil, aromatic mineral oil, synthetic liquid oligomers of polybutene, polypropene and polyterpene, isoparaffin oil, paraffin oil, or mixtures thereof. Most preferably, the liquid diluent is white petroleum mineral oil.

In the context of the present invention the term mineral oil such as white mineral oil also includes a technical grade mineral oil. Thus, the mineral oil may include small amounts of other chemicals. In a preferred embodiment, the purity of the mineral oil of the present invention is more than 90 %, preferably more then 95 %, even more preferably more than 98 %, most preferably more than 99 %, based on the total weight of the mineral oil.

A mineral oil of the present invention can be a mixture of liquid hydrocarbons. Mineral oils are generally obtained by refining crude petroleum oil. A mineral oil can comprise saturated hydrocarbons such as alkanes or unsaturated hydrocarbons such as alkenes or aromatic hydrocarbons. Preferably, the mineral oil comprises saturated hydrocarbons such as alkanes having 10 to 50, preferably 20 to 40 carbon atoms. Hydrocarbons include linear, branched or cyclic hydrocarbons.

A mineral oil of the present invention can have one or more of the physical properties such as a kinematic viscosity at 100 °C of 2 to 50 mm²/s, preferably 5 to 30 mm²/s as measured with a glass capillary viscometer, a specific gravity of 0.8 to 0.95, preferably of 0.82 to 0.9 and a boiling point of 200 to 400 °C, preferably of 250 to 350 °C

The liquid diluent is generally contained in the drug containing adhesive composition in an amount of less than 60 wt.%, preferably 15 to 50 % by weight, based on the total amount of the drug containing adhesive composition.

### Penetration enhancer:

The drug containing adhesive composition of the subject invention may further comprise one or more of a penetration enhancer (component d)). Since the skin presents a substantial barrier to ingress of foreign substances into the body, the art has recognized that the barrier to the transdermal delivery of an active ingredient through the skin can be overcome or reduced by incorporating excipients into the carrier that enhance the rate at which the active ingredient, i.e., the drug, passes, i.e., penetrates, through the skin. The term "enhancement" or "penetration enhancement" means an increase in the permeability of a biological membrane, such as the skin, to a drug, so as to increase the rate at which the drug permeates through the membrane and accelerate drug delivery.

An effective amount of penetration enhancer provides increased membrane permeability such as increased skin permeability, and thus provides increased rate of administration and amount of drug delivered through the skin. The term "penetration enhancer" relates to a substance or mixture of substances.

The penetration enhancers of the present invention include but are not limited to penetration enhancers selected from alkyl methyl sulfoxides, preferably decylmethyl sulfoxide, dimethyl sulfoxide; saturated fatty acids such as adipinic acid, caproic acid, caprylic acid, capric acid, myristic acid, lauric acid, stearic acid, palmitic acid, and alkyl esters thereof such as adipinic acid monoethylester; unsaturated fatty acids such as oleic acid, linoleic acid, linolenic acid, palmitoleic acid, and alkyl esters thereof, preferably oleyl oleate; saturated fatty alcohols such as myristyl alcohol, lauryl alcohol, stearyl alcohol, palmityl alcohol and cetyl alcohol; unsaturated fatty alcohols such as oleyl alcohol, palmitoleyl alcohol, elaidyl alcohol, linoleyl alcohol and linolenyl alcohol; azocyclo-alkan-2-ones, preferably 1-dodecylazacycloheptan-2-one; pyrrolidones such as 2-pyrrolidone, alkyl-2-pyrrolidone and N-methylpyrrolidone; glycols such as propylene glycol, polyethylene glycols, glycerol, dipropylene glycol, tripropylene glycol, diethylene glycol and triethylene glycol; alcohols, preferably ethanol, isopropyl alcohol, cyclohexanol; diethyltoluamide; tetrahydrofurfuryl alcohol; dimethyl formamide; dimethyl acetamide; 2,2,2-trichloroethanol; 2,2,2-trifluoroethanol; urea; salicylic acid; ethylene glycol monomethyl ether; N,N-dialkylhydroxylamine; 1,2-isopropylidene glycerol; N,N-dialkylnicotinamide; alkylaminooxide; hyaluronidase; isopropyl myristate; saccharose monooleate; lecithins; non-ionic surfactants; cholic acid; and derivatives thereof. Preferred penetration enhancers are alkylic fatty acid esters of saturated and/or unsaturated fatty acids, each containing from 8-18 carbon atoms, such as isopropylpalmitate or oleyl oleate (Cetiol^{®}) or isopropylmyristate.

The penetration enhancer is contained in the drug containing adhesive composition in an amount of 1 to 15 % by weight, preferably 3 to 7 % by weight, based upon the total weight of the drug containing adhesive composition.

### Tackifier:

The drug containing adhesive composition may further comprise one or more of a tackifier (component e)). Such tackifiers increase the adhesiveness of the adhesive layer.

Tackifiers which may be used in the drug containing adhesive composition according to the present invention include but are not limited to any compatible resins or mixtures thereof such as (1) natural or modified rosins, such as gum rosin, wood rosin, tall-oil rosin, distilled rosin, hydrogenated rosin, dimerized rosin, and polymerized rosin; (2) glycerol and pentaerythritol esters of natural or modified rosins, such as the glycerol ester of pale, wood rosin, the glycerol ester of hydrogenated rosin, the glycerol ester of polymerized rosin, the pentaerythritol ester of hydrogenated rosin, and the phenolic-modified pentaerythritol ester of rosin; (3) copolymers and terpolymers of natural terpenes, e.g., styrene/terpene and alpha methyl styrene/terpene; (4) polyterpene resins having a softening point, as determined by ASTM method E28,58T, of 80 to 150 °C.; the latter polyterpene resins generally resulting from the polymerization of terpene hydrocarbons, such as the bicyclic monoterpene known as pinene, in the presence of Friedel-Crafts catalysts at moderately low temperatures; also included are the hydrogenated polyterpene resins; (5) phenolic modified terpene resins and hydrogenated derivatives thereof, for example the resin product resulting from the condensation, in an acidic medium, of a bicyclic terpene and phenol; (6) aliphatic petroleum hydrocarbon resins having a Ball and Ring softening point of 70 to 135°C.; the latter resins resulting from the polymerization of monomers consisting of primarily of olefins and diolefins; also included are the hydrogenated aliphatic petroleum hydrocarbon resins; (7) alicyclic petroleum hydrocarbon resins and the hydrogenated derivatives thereof; (8) aliphatic/aromatic or cycloaliphatic/aromatic copolymers and their hydrogenated derivatives; and (9) styrene resins such as styrene-alpha-methylstyrene resin; or mixtures thereof.

Preferred tackifiers include but are not limited to tackifiers selected from polybutenes; polysiloxanes; elastomeric and polymeric resins; terpene-based esters such as from β-pines; aromatic, aliphatic and alkylaromatic resins; melamine formaldehyde resins; phenolic resins; hydroabietyl alcohol; synthetic resins; wood resins, preferably collophonium resin; or mixtures thereof.

In a further preferred embodiment, the tackifier is a resin, preferably selected from hydrogenated collophonium resin, styrene resin, hydrogenated petroleum hydrocarbon resin or a mixture thereof. The most preferred tackifier is a synthetic resin or wood resin such as collophonium ester, which can be hydrogenated. Hydrogenated collophonium resin is commercially available under the trademark Foral^{®}.

The one or more tackifier(s) are preferably contained in the drug containing adhesive composition in an amount of 10 to 70 % by weight, preferably 25 to 65 % by weight, more preferably 40 to 55 % by weight, based upon the total weight of the drug containing adhesive composition.

### Solubilizer and additives:

The drug containing adhesive composition may comprise further ingredients such as solubilizers, excipients, emollients, plasticizers, anti-irritants, opacifiers, fillers, antioxidants, as well as other components or additives that are commonly formulated into a drug containing adhesive composition.

The drug containing adhesive composition may thus comprise constituents such as solubilizers. Typically, the function of the solubilizer is such that it acts as a crystallization inhibitor and/or that it increases the mechanical stability of the transdermal therapeutic system. Such solubilizers further increase the solubility of the pharmaceutically active agent in the drug containing adhesive composition.

The solubilizer might be present in the drug containing adhesive composition in an amount of 1 to 30 % by weight, preferably 5 to 15 % by weight, based upon the total weight of the composition.

Solubilizers which may be used in the drug containing adhesive composition of the transdermal therapeutic system according to the present invention include but are not limited to soluble polyvinyl pyrrolidones as commercially available under the trademark Kollidon®. A soluble polyvinyl pyrrolidone is generally obtained by radical polymerization of N-vinyl pyrrolidone. Soluble polyvinyl pyrrolidone is also known as povidon(e), povidonum, polyvidone, poly(l-vinyl-2-pyrrolidone) and PVP.

Preferably, the solubilizer is a (preferably non-crosslinked) polyvinyl pyrrolidone having a weight average molecular weight Mw of 1,000 to 3,000,000, more preferably from 100,000 to 2,000,000, most preferably from 1,000,000 to 1,500,000 as measured by gel permeation chromatography.

The molecular weight of polyvinyl pyrrolidone (povidone) is usually expressed as the K-value. The polyvinyl pyrrolidone of the present invention preferably has a K-value of 10 to 100, most preferably from 80 to 95.

Further examples of such solubilizers include, but are not limited to, cyclodextrins and cyclodextrin derivatives such as substituted cyclodextrins; 2-(2-ethoxyethoxy)ethanol, urea, methyl 2-methylprop-2-enoate, neohesperidine, alcohols and polyols such as ethanol, isopropanol, butanol, benzyl alcohol, ethylene glycol, propylene glycol, octyldecanol, octyldodecanol, butanediols and isomers thereof, glycerol, pentaerythritol, sorbitol, mannitol, dimethyl isosorbide, polyethylene glycol, polypropylene glycol, polyvinylalcohol; hydroxypropyl methylcellulose and other cellulose derivatives; ethers of polyethylene glycols (PEG) having an average molecular weight of about 200 to about 6000, such as tetrahydrofurfuryl alcohol PEG ether (glycofurol) or methoxy PEG; amides such as 2-pyrrolidone, 2-piperidone, s-caprolactam, N-alkylpyrrolidone, N-hydroxyalkyl pyrrolidone, N-alkylpiperidone, N-alkylcaprolactam, dimethylacetamide, and polyvinyl pyrrolidone; esters, such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, p-butyrolactone and isomers thereof; and other solubilizers known in the art, such as Eudragit® E100, dimethyl acetamide, dimethyl isosorbide, N-methyl pyrrolidones, monooctanoin, diethylene glycol monoethyl ether, and water. Mixtures of solubilizers are also within the scope of the invention. Preferred solubilizers include triacetin, triethylcitrate, ethyl oleate, ethyl caprylate, dimethylacetamide, N-methylpyrrolidone, N-hydroxyethylpyrrolidone, hydroxypropyl methylcellulose, hydroxypropyl cyclodextrins, ethanol, glycofurol, diethylene glycol monoethyl ether, propylene glycol, dimethyl isosorbide and polyvinyl pyrrolidone. Particularly preferred solubilizers include sorbitol, glycerol, triacetin, ethyl alcohol, polyethylene glycol, glycofurol, propylene glycol and polyvinyl pyrrolidone, most preferably polyvinyl pyrrolidone.

The drug containing adhesive composition may also further comprise fillers. Fillers which may be used in the drug containing adhesive composition according to the present invention include but are not limited to silicon dioxide, metal oxides such as titanium dioxide or zinc dioxide, talc, silicates such as magnesium silicate or aluminium silicate, stearates, such as zinc stearate, or mixtures thereof.

Fillers may be contained in the drug containing adhesive composition in an amount from 0 to 30 wt.%, preferably from 5 to 20 % by weight, based on the total weight of the drug containing adhesive composition.

The drug containing adhesive composition may further comprise one or more of an antioxidant. Preferred antioxidants are selected from hindered phenols such as 1,3,5-trimethyl 2,4,6-tris (3,5-di-tert-butyl-4-hydroxybenzyl) benzene; pentaerythrityl tetrakis-3(3,5-di-tert-butyl-4-hydroxyphenyl)-propionate; 4,4'-methylenebis (2,6-tert-butylphenol); 4,4'-thiobis (6-tert-butyl-o-cresol); 2,6-di-tert-butylphenol; 6-(4-hydroxy-phenoxy)-2,4-bis(n-octylthio)-1,2,5-triazine; di-n-octadecyl 3,5-di-tert-butyl-4-hydroxybenzyl phosphonate; 2-(n-octylthio)ethyl 3,5-di-tert-butyl-4-hydroxybenzoate; and sorbitol hexa[3-(3,5-di-tert-butyl-4-hydroxyphenyl)-propionate], or mixtures thereof. The one or more antioxidants are typically comprised in the drug containing adhesive composition in an amount up to 5 wt.%, preferably from 0.3 to 3 % by weight, based on the total weight of the drug containing adhesive composition.

### Transdermal therapeutic systems (TTS):

A transdermal therapeutic system according to the present invention typically comprises a backing layer, at least one drug containing adhesive layer and a release liner. The at least one drug containing adhesive layer(s) is formed from the drug containing adhesive composition described above and thus comprises or consists of the drug containing adhesive composition. In a preferred embodiment, the drug containing adhesive layer consists of the drug containing adhesive composition. The transdermal therapeutic systems of the present invention are often referred to as matrix-controlled transdermal therapeutic systems or matrix TTS.

The TTS of the present invention typically comprise a backing layer, which usually is impermeable for the pharmaceutically active agent. The TTS of the present invention may further comprise one or more additional drug containing adhesive layers. The TTS may further comprise one or more further adhesive layers, which do not contain an active ingredient. All embodiments described above for the drug containing adhesive composition also apply for adhesive compositions, which are used for forming the further adhesive layer(s). The weight percentages are then to be based on a composition containing the ingredients b) to d) and optionally e). The adhesive layer(s) thus may form one or more separate layers or be part of the drug containing layer(s). On the side, which is opposite to the backing layer the TTS generally comprises a release liner such as a peelable release liner.

Preferably, the drug containing adhesive layer is, on one side, affixed to the backing layer, and, on another side, to the release liner thus providing for a multi-layered design.

### Backing layer:

The backing layer is typically impermeable to the drug or other excipients. The backing layer is thus generally made of a material that is impermeable to the pharmaceutically active agent and other excipients of the matrix layer. The backing layer serves as a protective cover for the matrix layer and provides a support function. The backing layer can be formed so that it is essentially the same size as the matrix layer containing the pharmaceutically active agent. The backing layer can be of any appropriate thickness that will provide the desired protective and support functions. A suitable thickness is from about 5 µm to about 300 µm. More specifically, the thickness is less than about 150 µm, yet more specifically, it is less than about 100 µm, and most specifically, the thickness is less than about 50 µm. The thickness is further preferably more than 5 µm, more preferably more than 10 µm.

Examples of materials suitable for making the backing layer are films of acrylate, acrylonitrile-butadiene-styrene, acrylonitrile (methyl methacrylate) copolymer, acrylonitrile copolymer, ethylene ethyl acrylate, ethylene methyl acrylate, ethylene vinyl acetate, ethylene vinyl acetate copolymer, ethylene vinyl alcohol polymer, ionomers, nylon (polyamide), nylon (polyamide) copolymer, polybutylene, polycarbonate, polyester, polyethylene terephthalate (PET), thermoplastic polyester copolymer, polyethylene copolymer (high density), polyethylene (high-molecular-weight, high density), polyethylene (intermediate-molecular weight, high density), polyethylene (linear, low density), polyethylene (low density), polyethylene (medium density), polyethylene oxide, polyimide, polypropylene, polypropylene (coated), polypropylene (oriented), polystyrene, polyurethane, polyvinyl acetate, polyvinyl chloride, polyvinylidene chloride and/or styrene-acrylonitrile. It is within the present invention that such films may be metallised or pigmented.

Preferred materials for the manufacture of the backing layer are polyurethane, ethylene vinyl alcohol polymer and polyester, most preferably polyethylene terephthalate (PET).

In a preferred embodiment the backing layer has a tensile strength in the longitudinal and in the cross direction of 100 to 500 N/mm², preferably of 200 to 300 N/mm², most preferably of 220 to 280 N/mm² as measured by ISO 527-1 and 527-3 using test piece type 2, test speed 100%/min; 23 °C, 50 % rel. humidity.

In a further preferred embodiment the backing layer has an elongation at break in the longitudinal direction of 100 to 200 %, preferably of 105 to 150 %, and in the cross direction of 100 to 120 %, most preferably of about 100 % as measured by ISO 527-1 and 527-3 using test piece type 2, test speed 100%/min; 23 °C, 50 % rel. humidity.

In a further preferred embodiment the backing layer has an elasticity modulus in the longitudinal and in the cross direction of 2,000 to 10,000 N/mm², preferably of 4,000 to 6,000 N/mm² as measured by ISO 527-1 and 527-3 using test piece type 2, test speed 100%/min; 23 °C, 50 % rel. humidity.

Moreover, the backing layer preferably provides gas barrier properties as determined with a layer of 12 µm thickness. For air the gas barrier is preferably 20 to 100, more preferably of 40 to 80 cm³/m²*d*bar as measured by DIN 53380 at 23 °C, 0 % rel. humidity. For oxygen the gas barrier is preferably 50 to 150, more preferably of 80 to 130 cm³/m²*d*bar as measured by DIN 53380 at 23 °C, 50 % rel. humidity. For water vapour the gas barrier is preferably 5 to 150, more preferably of 10 to 250 g/m²*d as measured by DIN 53122 at 23°C, 85 % rel. humidity. For nitrogen the gas barrier is preferably 10 to 100, more preferably of 20 to 45 cm³/m²*d*bar as measured by DIN 53380 at 23 °C, 0 % rel. humidity. For carbon dioxide the gas barrier is preferably 100 to 5000, more preferably of 250 to 750 cm³/m²*d*bar as measured by DIN 53380 at 23°C, 0 % rel. humidity.

### Release liner:

The release liner, which preferably is a peelable release liner, is to protect the adhesive, which mediates the attachment of the transdermal therapeutic system to the subject to which the TTS is applied. The release liner is thus to be removed from the transdermal delivery system prior to the application to the subject.

Release liners can be formed of polyester, polyethylene, polypropylene, polysiloxane, e.g. with a fluorosiliconized coating, polyacrylate, ethylene vinyl acetate, polyurethane, polyisobutene or paper. Preferably the paper is coated with silicone and/or polyethylene. In an embodiment, a foil consisting of polyethylene terephthalate (PET) is used, whereby, preferably, one side of such foil is siliconized. Typically, the thickness of such release liner is from 50 to 120 µm, preferably from 60 to 100 µm. Also a combination of any of the above materials may be used in the preparation of the release liner. The release liner preferably also comprises an adhesive, which may be one as defined herein.

Moreover, the release liner can be made from the same material as the backing layer. In this embodiment the features described above for the backing layer equally apply for the release liner.

### Manufacture:

The TTS according to the present invention may be manufactured by preparing a solution or dispersion of the ingredients as described above, coating the solution or dispersion on the release liner, drying the coating to form the adhesive layer, and then optionally coating the adhesive layer with a backing layer. The features as described above for the transdermal therapeutic system and the drug containing adhesive composition also apply for the manufacture of a TTS according to the present invention

In the method of manufacture according to the present invention, the drug containing adhesive composition forming the drug containing adhesive layer is generally prepared in a first step by mixing fentanyl (a), the styrenic polymer (b), liquid diluent (c), penetration enhancer (d) and optionally tackifier (e) or a further additive in an appropriate solvent. The ingredients are mixed, for example by mechanically stirring the mixture. Stirring is preferably achieved until the polymer is completely solved under visual inspection.

Suitable solvents include, but are not limited to, organic solvents such as ethanol, ethyl acetate, 2-propanol, heptane, hexane, isopropyl alcohol, methanol, toluene, 2,4-pentandion and mixtures thereof. The drug containing solution or dispersion (in the following abbreviated as solution/dispersion) is typically mixed at a temperature between 20 °C and 25 °C. Homogenization can be achieved by using a stirrer such as a magnetic stirrer.

In a further embodiment the drug and optional excipients are added to a suitable solvent and mixed to form a first solution/dispersion. A second solution/ dispersion comprising the styrenic polymer is then added to the first solution/ dispersion. The thus obtained mixture of the first and second solution/ dispersion is then further homogenized until the styrenic polymer and preferably all ingredients are solved to form a solution.

The solution or dispersion is then preferably coated on a release liner. Preferably, the solution/dispersion is applied to a siliconized side of a release liner. Such a release liner e.g. consists of a transparent foil such as a PET-foil as explained above. The coating is dried to remove the solvent partially or completely, preferably the solvent is removed completely, and to form the drug containing adhesive layer.

In a further embodiment, the release liner is first coated with one or more adhesive layers, which do not contain an active ingredient, wherein the one or more adhesive layers are then coated with the drug containing adhesive layer of the invention.

The dried adhesive layer(s) are then supplied with a backing layer on the side, which is opposite to the release liner.

Alternatively, the drug containing homogenous solution is applied to a film, which acts as a backing layer e.g. a PET film such as Hostaphan^{®} RN 19 is used as the backing layer, preferably a drug impermeable backing layer. The thus one-side coated backing layer is then usually dried as described above. Upon drying, the drug containing adhesive layer is formed from the drug containing adhesive composition. The dried adhesive layer is then supplied with a release liner. The transdermal patches are subsequently punched from the thus obtained layered product.

The invention thus further refers to the transdermal therapeutic system obtainable by the above described methods of manufacture. All preferred embodiments and features as described above for the transdermal therapeutic system of the present invention thus also apply for the methods of manufacture of the TTS as described herein and the TTS obtainable by these methods.

The TTS of the present invention preferably is 1 to 200 cm² in size, more preferably 5 to 70 cm². The coating weight of the drug containing adhesive layer per unit area is typically between 40 and 150 g/m², preferably between 50 and 100 g/m², most preferably 60 to 80 g/m².

In a preferred embodiment, the transdermal therapeutic system comprises an adhesive layer containing an amount of fentanyl sufficient to maintain a constant release of the fentanyl or fentanyl analogue through the skin of a patient for the entire period of use of the TTS.

The transdermal therapeutic system of the invention provides delivery of fentanyl to a patient in need of the active ingredient. On administration over skin the TTS generally exhibits a steady state drug flux of 0.1 to 20 µg/cm²/h, preferably 0.8 to 10 µg/cm²/h and more preferably 1 to 3 µg/cm²/h. Steady state administration rates obtainable according to this invention range 12 to 150 µg/h.

Preferably, the flux remains constant for period of at least 24 h, more preferably for at least 72 h, most preferably for at least 96 h. In addition, the fentanyl flux generally should remain constant up to 120 h, more preferably for up to 144 h, most preferably up to 168 h. It is particularly preferred that the transdermal fentanyl flux is between 0.8 to 10 µg/cm²/h for at least 72 h.

The TTS thus percutaneously releases the active ingredient in an amount, which is sufficient to induce analgesia. Preferably, the TTS of the present invention releases fentanyl in an amount sufficient to obtain a constant blood plasma level of 10 to 150 pg/cm²/h, more preferably of 20 to 100 pg/cm²/h. Blood plasma levels are determined as an average of at least 12 patients as described in "Bauer, Frömming, Fuhrer, 8^{th} edition, 2006, Wissenschaftliche Verlagsgesellschaft mbH, Stuttgart, chapter 4.2.1". Fentanyl is preferably released to the patient to achieve a constant blood plasma level for the entire period of administration, which generally is more then 24 hours, preferably more then 72h. The TTS of the invention may be used to achieve a constant blood plasma level of fentanyl up to 4 days, preferably up to 7 days.

The transdermal therapeutic system is used for treating analgesia in a patent. In particular, the transdermal therapeutic system is used for treating a patient suffering from acute or chronic pain.

The invention will be described further in the following examples, which are included for purposes of illustrating the invention and are not intended, in any way, to be limiting of the scope of invention.

### EXAMPLES

### Example 1: Manufacture of a transdermal therapeutic system

85.3 g of a styrenic adhesive solution comprising a blend of hydrogenated styrenic block copolymers, synthetic tackifier resins and white mineral oil as liquid diluent and having a polymer content of 60.5 wt.% in toluol and heptane as solvents, 3 g hydrogenated collophonium resin (Foral^{®} 105 E), 3 g isopropyl palmitate, 24 g ethyl acetate and 2.4 g fentanyl base were homogenized. The mixture was stirred for about 1 h and controlled visually, whether all solids were solved. The solution was then coated on a transparent polyester foil of a width of 280 mm, resulting in a coating weight per unit area of about 60 g/m². The siliconized polyester layer served as a release liner. The solvents were removed by drying with heated air, which was streamed over the wet coating. The adhesive coating was then covered with a polyester foil of a thickness of 15 µm. An area of 10 cm² was then cut by appropriate cutting tools. The edges between distinct systems were removed.

### Example 2: Manufacture of a transdermal therapeutic system

80.3 g of a styrenic adhesive solution comprising a blend of hydrogenated styrenic block copolymers, synthetic tackifier resins and white mineral oil as liquid diluent and having a polymer content of 60.5 wt.% in toluol and heptane as solvents, 3 g hydrogenated collophonium resin (Foral^{®} 105 E), 6 g soy bean oil, 24 g ethyl acetate and 2.4 g fentanyl base were homogenized. The mixture was stirred for about 1 h and controlled visually, whether all solids were solved. The solution was then coated on a transparent polyester foil of a width of 280 mm, resulting in a coating weight per unit area of about 80 g/m². The siliconized polyester layer served as a release liner. The solvents were removed by drying with heated air, which was streamed over the wet coating. The adhesive coating was then covered with a polyester foil of a thickness of 15 µm. An area of 10 cm² was then cut by appropriate cutting tools. The edges between distinct systems were removed.

### Example 3: Evaluation of fentanyl skin permeation

The in vitro skin permeation of fentanyl through human skin was evaluated using the following compositions (all percentages are weight percentages based on the total amount of the compositions):

| | | **Test 1** | **Test 2 (reference)** |
|---|---|---|---|
| Fentanyl | | 4% | 8% |
| Adhesive | | styrenic polymer* | acrylic polymer, Durotak^{®} 87-4287 |
| Tackifier | Hydrogenated collophonium resin (Foral^{®} 105 E) | 5% | 0% |
| Enhancer | Isopropyl myristate | 5% | 0% |

| | | | |
|---|---|---|---|
| * as described in Example 1 | | | |

Reference test was a Durogesic^{®} patch containing 8 % fentanyl in a polyacrylate adhesive (Durotak^{®} 87-4287). The fentanyl patch Test 1 was prepared as described above for Example 1.

The flux was determined by using a two-compartment diffusion cell with a section of human epidermis mounted between the cell halves. A TTS was adhered to one side of the skin and a drug-receiving medium was placed on the receptor-side of the cell. The apparatus was placed in a water bath maintained at 32±1 °C. Samples of the receptor medium were collected over a period of 96 hours for HPLC analysis of drug concentration.

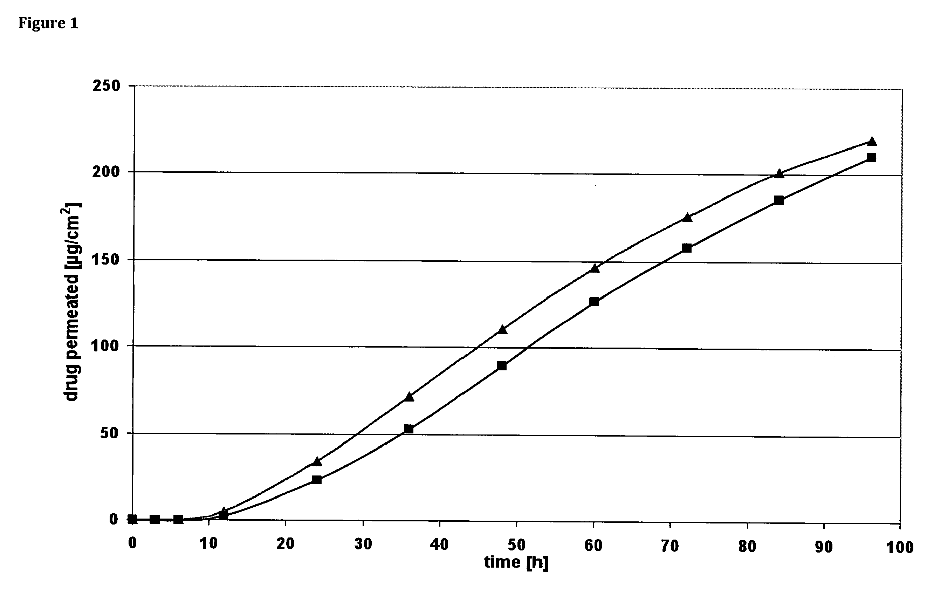

It can be seen by Figure 1 that the TTS according to the invention (Test 1, triangles) containing 4 wt.% fentanyl in combination with a styrenic polymer adhesive shows increased fentanyl in vitro skin flux compared to the reference TTS containing twice the amount of fentanyl (8 wt.%) but in combination with an acrylic polymer adhesive (Test 2, squares). Thus, it was surprisingly found by the inventors that when using a styrenic polymer adhesive, high fentanyl flux can be achieved even in case of a low dose fentanyl patch.

### Example 4: Evaluation of blood plasma levels after transdermal delivery of fentanyl

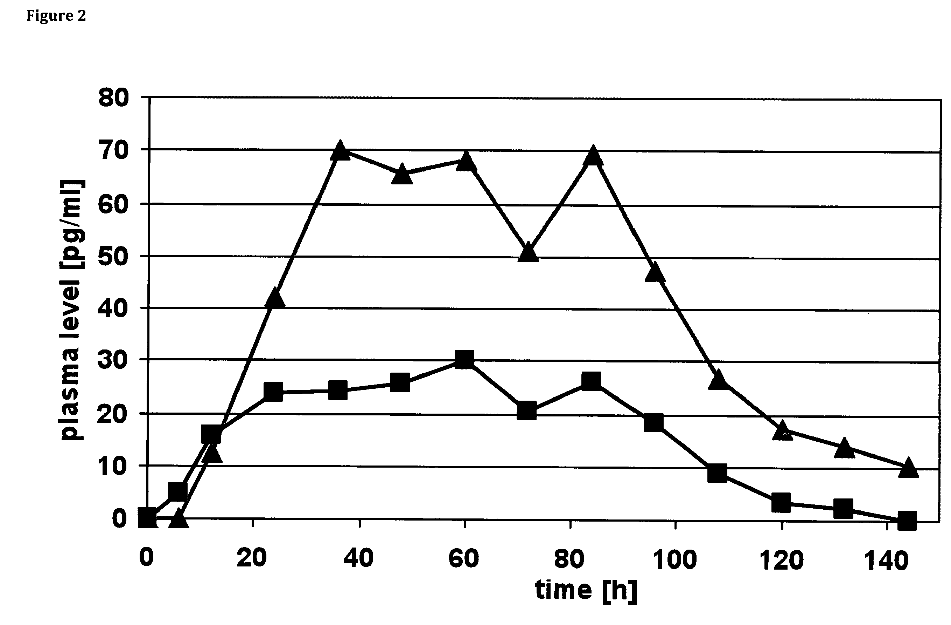

The study was a single-dose, four-treatment, one-period parallel study. 12 female minipigs (test species: Göttingen Minipigs) were allocated to one of 2 test groups employing a pseudo-random body weight stratification procedure. The patches were applied to the left or right flank and removed 96 hours after application. Blood samples were collected in blood collection tubes before patch application and at the following times thereafter: 6, 12, 24, 36, 48, 60, 72, 84, 96, 108, 120, 132 and 144 hours. Blood samples were analyzed for fentanyl concentration levels and shown in Table 1 below and in Figure 2.

Test 1 and Test 2 (reference) were as described above.

**Table 1:**

| | **Test 1, animals 1-6** | **Test 2 (reference), animals 7-12** |
|---|---|---|
| Time [h] | c_{fen} [pg/ml] | c_{fen} [pg/ml] |
| 0 | 0 | 0 |
| 6 | 0 | 4.8 |
| 12 | 13 | 16 |
| 24 | 42 | 24 |
| 36 | 70 | 24 |
| 48 | 66 | 26 |
| 60 | 68 | 30 |
| 72 | 51 | 21 |
| 84 | 70 | 26 |
| 96 | 47 | 18 |
| 108 | 27 | 8.8 |
| 120 | 17 | 3.4 |
| 132 | 14 | 2.0 |
| 144 | 10 | 0 |

It can be seen by Figure 2 that the TTS according to the invention (Test 1, triangles) leads to increased blood plasma concentrations of fentanyl compared to the comparative TTS (Test 2, squares). In particular, the TTS of the invention releases fentanyl in an amount sufficient to obtain a blood plasma level of at least 40 pg/ml over a period of more than 72 h. The reference composition of Test 2 comprising an acrylic polymer as adhesive shows significantly reduced blood plasma concentrations of 30 pg/ml and below. Thus, it was surprisingly found by the inventors that when using a styrenic polymer adhesive, a high blood plasma concentration of fentanyl can be achieved even in case of a low dose fentanyl patch containing only 4 % of fentanyl.

In summary, the invention provides a transdermal system having one or more of the benefits of
(1) a long lasting (three days or more) and continuous release of active ingredient,
(2) good tolerability by the skin over the entire period of use,
(3) reliable efficacy,
(4) preventing abuse of the drug contained in the transdermal system,
(5) good comfort even after the entire period of use,
(6) inexpensive production with a highly reproducibly quality,
(7) the dosage can be adjusted by simply varying the size of the skin-contact area,
(8) high stability in storage, and
(9) high mechanical stability due to the homogeneous structure of the system.

## Claims

1. A transdermal therapeutic system comprising
1) a backing layer,
2) a drug containing adhesive composition,
3) optionally one or more adhesive layers, and
4) further optionally a release liner,
wherein the drug containing adhesive composition comprises
a) 0.1 to 6.0 wt.% of fentanyl, based on the total amount of the composition,
b) a styrenic polymer, preferably a styrenic copolymer,
c) a liquid diluent,
d) a penetration enhancer, and
e) optionally a tackifier.

2. The transdermal therapeutic system of claim 1, wherein the styrenic polymer is an A-B-A triblock copolymer, which preferably comprises at least one styrenic polymer block A, preferably polystyrene, and at least one polymer block B obtained from monomers selected from ethylene, propylene, isoprene, butylene, butadiene, or mixtures thereof.

3. The transdermal therapeutic system of claim 2, wherein the A-B-A triblock copolymer is selected from styrene-ethylene-styrene (SES), styrene-butadiene-styrene (SBS), styrene-isoprene-styrene (SIS), styrene-ethylene/butylene-styrene (S/EB/S), styrene-ethylene/butylene/propylene-styrene (S/EBP/S), styrene-isoprene/butadiene-styrene (S/IB/S), or mixtures thereof.

4. The transdermal therapeutic system of claim 2 or 3, wherein the glass transition temperature Tg as measured by ISO 11357-1, -2, -3 of the A-block is higher than the glass transition temperature of the B-block.

5. The transdermal therapeutic system of anyone of claims 2 to 4, wherein the glass transition temperature Tg as measured by ISO 11357-1, -2, -3 of the A-block is 20 to 150 °C and/or the glass transition temperature of the B-block is -25 to 10 °C.

6. The transdermal therapeutic system of any one of the previous claims, wherein the drug containing adhesive composition further comprises a styrenic A-B diblock copolymer, wherein blocks A and B are as defined in anyone of claims 2 to 5 and which preferably is selected from styrene-ethylene (SE), styrene-butadiene (SB), styrene-isoprene (SI), styrene-ethylene/butadiene-styrene (SE/BS), styrene-ethylene/propylene (S/EP), or mixtures thereof.

7. The transdermal therapeutic system of any one of the previous claims, comprising 1 to 5 wt.%, preferably 2.5 to 4.0 wt.% of fentanyl, based on the total amount of the drug containing adhesive composition.

8. The transdermal therapeutic system of any one of the previous claims, wherein the liquid diluent is contained in the drug containing adhesive composition in an amount of less than 60 wt.%, preferably 15 to 50 wt.%, based on the total amount of the drug containing adhesive composition.

9. The transdermal therapeutic system of any one of the previous claims, wherein the liquid diluent is mineral oil, preferably paraffinic mineral oil, naphthenic mineral oil, aromatic mineral oil, or mixtures thereof, most preferably white petroleum mineral oil.

10. The transdermal therapeutic system of any one of the previous claims, wherein the penetration enhancer is an alkylic fatty acid ester of saturated and/or unsaturated fatty acids containing from 8-18 carbon atoms, preferably selected from isopropyl palmitate, isopropyl myristate, or a mixture thereof.

11. The transdermal therapeutic system of any one of the previous claims, wherein the penetration enhancer in contained in the drug containing adhesive composition in an amount of 1 to 15 wt.%, preferably 3 to 7 wt.%, based on the total amount of the drug containing adhesive composition.

12. The transdermal therapeutic system of any of the previous claims, wherein the tackifier is a resin, preferably selected from hydrogenated collophonium resin, styrene resin, hydrogenated petroleum hydrocarbon resin, or mixtures thereof.

13. The transdermal therapeutic system of any one of the previous claims, wherein the tackifier is contained in the drug containing adhesive composition in an amount of 10 to 70 wt.%, preferably 25 to 65 wt.%, based on the total amount of the drug containing adhesive composition.

14. A method of producing a transdermal therapeutic system comprising a backing layer, at least one adhesive layer and a release liner, comprising the steps of
1) preparing a solution or dispersion comprising
a. fentanyl,
b. a styrenic polymer, preferably a styrenic copolymer, and
c. optionally one or more of a penetration enhancer, liquid diluent or tackifier,
2) coating the solution or dispersion on the backing layer or on the release liner,
3) drying the solution or dispersion to form the at least one adhesive layer, and
4) coating the at least one adhesive layer with the release liner or the backing layer.

15. A transdermal therapeutic system obtainable by the method of claim 14.
